(19) Europäisches Patentamt
European Patent Office
Office européen des brevets

(11) Numéro de publication : **0 238 419 B1**

(12) **FASCICULE DE BREVET EUROPEEN**

(45) Date de publication du fascicule du brevet :
**16.10.91 Bulletin 91/42**

(21) Numéro de dépôt : **87420004.1**

(22) Date de dépôt : **07.01.87**

(51) Int. Cl.⁵ : **A61M 5/14, A61B 17/34**

(54) **Canule pour ponction et son procédé de fabrication.**

(30) Priorité : **10.01.86 DE 3600496**

(43) Date de publication de la demande :
**23.09.87 Bulletin 87/39**

(45) Mention de la délivrance du brevet :
**16.10.91 Bulletin 91/42**

(84) Etats contractants désignés :
**AT BE CH DE ES FR GB IT LI LU NL SE**

(56) Documents cités :
**EP-A- 0 091 227**
**EP-A- 0 158 697**
**DE-A- 2 259 865**
**FR-A- 2 507 898**

(73) Titulaire : **HOSPAL AG**
**Missionsstrasse 60/62**
**CH-4012 Basel (CH)**

(72) Inventeur : **Magasi, Josef**
**Wendelinusstrasse 8**
**W-6902 Sandhausen (DE)**

(74) Mandataire : **Kerneis, Daniéle et al**
**Hospal COT Service Brevets P.O. Box 21**
**F-69881 Meyzieu Cédex (FR)**

## Description

La présente invention concerne une canule pour ponction et son procédé de fabrication.

De telles canules sont par exemple utilisées en médecine humaine pour réaliser des ponctions effectuées dans une cavité normale ou pathologique, à travers les tissus qui la recouvrent, pour en retirer du liquide ou y introduire un médicament.

On peut ainsi réaliser une ponction intra-musculaire dans le but d'injecter un anti-inflammatoire par exemple.

On utilise également de telles canules pour effectuer une ponction directe de fistules artério-veineuses suivant la technique de Brescia et Cimino ou pour la ponction de vaisseaux plus importants pour des hémodialyses chroniques ou pour le prélèvement d'assez grandes quantités de sang.

D'autre part, lors d'une ponction biopsie, la ponction est réalisée pour prélever un fragment de tissu, en vue d'un examen au microscope.

Bien que les canules, objets de la présente invention puissent être utilisées pour toutes sortes de ponctions, la description qui va suivre se rapporte plus particulièrement à la ponction de vaisseaux.

De telles canules sont décrites par exemple dans les mémoires descriptifs des demandes de brevets allemands DE-A-2 259 865 et DE-A-2 259 866.

Elles comportent une surface tranchante polie plane pour la ponction percutanée des vaisseaux, ce qui permet de réduire largement le traumatisme des tissus, mais provoque par contre une ouverture importante en rapport avec la paroi de la canule, car on doit atteindre 300 ml de sang par minute avec une vitesse d'écoulement de 4 m/s.

Les canules connues présentent un biseau et en général une ouverture dans le fond assez près du biseau, grâce à quoi on peut éviter que la paroi du vaisseau soit plaquée par aspiration sur l'ouverture du biseau.

On ponctionne des patients avec ces canules plus de 100 fois par an pour effectuer une hémodialyse, mais on ne dispose que de surfaces vasculaires relativement petites.

Au moment de la piqûre avec des canules telles que décrites ci-dessus, il se forme une coupure en arc. Malheureusement, il arrive à plusieurs reprises que le lobe ainsi formé soit entraîné au moment de la piqûre par l'arête supérieure opposée à la pointe de la canule et pénètre dans l'incision. La cicatrice et l'induration consécutives à la cicatrisation qui doit être très rapide, gênent une nouvelle ponction dans cette zône de tissus.

Le document EP-A-0 158 697 décrit une aiguille comprenant une avancée qui possède des bords coupants et qui se termine par une pointe effilée ; la pointe située sur l'axe de l'aiguille est reliée au corps de l'aiguille par 2 passerelles opposées délimitant chacune 2 ouvertures. Ainsi, cette aiguille s'ouvre sur l'extérieur par 4 ouvertures, chacune d'elle étant bordée d'un côté par une passerelle et de l'autre côté par un bord coupant de l'avancée.

Cette aiguille présentée comme résolvant un certain nombre des problèmes de l'art antérieur, semble cependant difficile à produire.

Elle présente également l'inconvénient d'être fragile par manque de rigidité et de ne pas présenter suffisamment de surface d'appui lors de la pénétration de l'aiguille à l'intérieur du matériau à ponctionner. Le nombre élevé d'ouvertures risque en outre de provoquer des turbulences pouvant induire une gêne chez le patient.

Pour pallier à ces inconvénients, la présente invention propose une canule pour ponction comprenant un corps tubulaire dont l'une des extrémités comporte une avancée se terminant par une pointe permettant d'inciser le matériau à ponctionner, la pointe étant reliée au corps tubulaire par une passerelle constituant une surface lisse de guidage du matériau ponctionné vers le corps tubulaire, caractérisée en ce que la portion du corps tubulaire opposée à ladite passerelle est un secteur plein coopérant avec cette passerelle pour définir 2 ouvertures situées de part et d'autre de la passerelle.

Cette passerelle permet au moment de l'introduction de la canule de relever le lobe en forme d'arc mentionné ci-dessus, de le soutenir et d'éviter ainsi qu'il soit introduit dans l'incision.

La passerelle forme ainsi une surface de guidage pour assurer le déplacement du matériau ponctionné vers la section tubulaire, lors de l'introduction de la canule.

De façon préférentielle, la passerelle est infléchie vers l'intérieur de la canule de telle façon que son extrémité soit raccordée tangentiellement à la pointe de la canule.

Selon la présente invention, on forme de chaque côté de la passerelle des ouvertures allongées, pointues vers l'avant et arrondies vers l'arrière, formant un angle entre elles.

Ceci permet d'éviter une ouverture supplémentaire dans le fond de la canule.

De plus, quand on utilise la canule selon l'invention pour injecter un médicament par ponction intramusculaire par exemple, la présence d'ouvertures latérales permet une bonne distribution du liquide injecté.

Le biseau de la pointe de la canule n'atteint en général pas le diamètre complet car il s'arrête avant. De ce fait, on utilise l'élasticité du tissu pour maintenir la taille de l'incision aussi petite que possible. Le tissu est distendu par la zône voisine de l'arête coupante et est poussé sur la canule. Pour assurer cette distension, tout en évitant un rabotage des bords de la plaie, on prévoit selon l'invention une taille en biais des ouvertures.

Selon la présente invention, on fabrique les nouvelles canules de la façon suivante : on exerce sur l'arête antérieure d'un matériau pour canule de forme tubulaire une force ponctuelle ou longitudinale, on enfonce ainsi le tube jusqu'à ce qu'il soit en contact avec le point radialement opposé du tube, on procède éventuellement à sa fixation puis à une taille permettant de former une passerelle s'étendant à partir d'une pointe de l'aiguille vers l'arrière avec deux ouvertures latérales.

Par ailleurs, les ouvertures peuvent être taillées en biais et la partie non enfoncée du tube peut être taillée en arêtes coupantes se terminant en pointe d'une manière connue en soi, la distance entre les arêtes coupantes à l'endroit le plus large étant plus petite que le diamètre externe du tube.

Selon un mode préférentiel de réalisation la passerelle est en matière plastique. Selon un autre mode préférentiel de réalisation, l'ensemble de la tête de la canule conforme à la présente invention est réalisé en matière plastique. En règle générale, la canule de ponction selon la présente invention est réalisée en matériau inoxydable ou en alliage de métal inoxydable, par exemple en acier inoxydable.

La présente invention sera mieux comprise à l'aide de la description qui va suivre, en référence aux dessins annexés qui illustrent, de façon schématique et sans échelle déterminée un mode de réalisation de la présente invention.

La figure 1 représente une vue latérale d'une canule selon la présente invention.

La figure 2 est une vue de face de ladite canule.

Les figures 3 et 4 représentent des étapes de fabrication de la pointe de la canule.

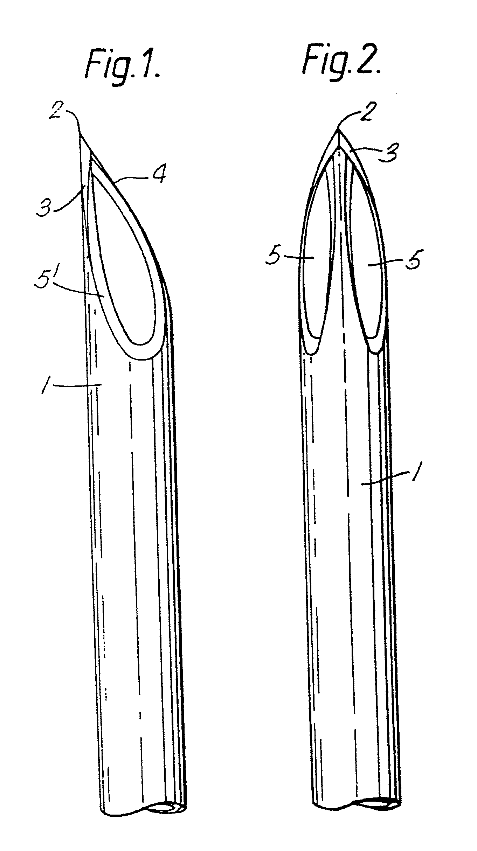

Les figures 1 et 2 représentent le corps de la canule 1. La passerelle 4 fait saillie vers l'arrière à partir de la pointe 2 pour former une seule pièce avec la paroi de la canule.

Le biseau 3 aboutit à la pointe 2 ; il se termine avant d'atteindre le diamètre externe de la canule et aboutit dans la zone périphérique des ouvertures 5. Ces ouvertures latérales 5 possèdent une taille en biais 5', qui se recoupe partiellement avec le biseau 3 de la pointe de la canule, comme on le voit clairement sur la vue latérale de la figure 1. De façon préférentielle, l'extrémité de la passerelle (4) est collée ou soudée à la surface adjacente à la pointe (2) de la canule par tout moyen connu en soi, par exemple au laser. Afin de réaliser une passerelle renforcée, on utilise, de préférence pour la fabrication de la canule un matériau en V. La canule est polie intérieurement et extérieurement et recouverte de silicone.

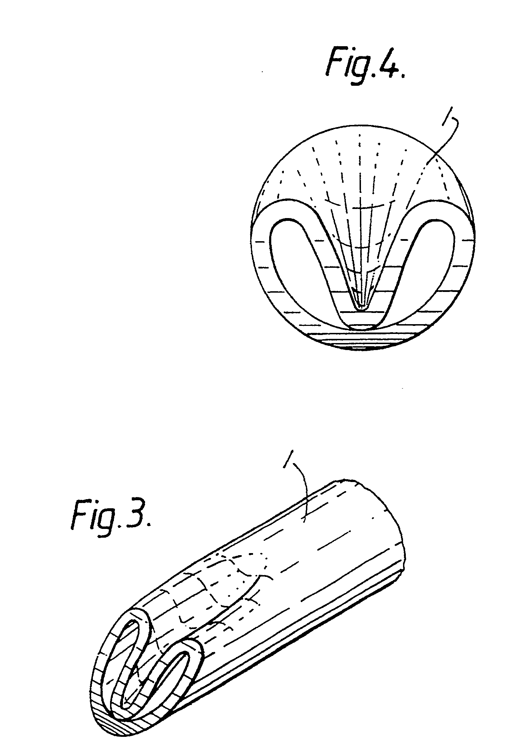

La figure 3 est une représentation en perspective de la canule avec sa partie frontale enfoncée, conformément à la présente invention.

La figure 4 est une vue en plan de cette partie frontale, ainsi que de la coupe en forme de V et des arêtes coupantes.

## Revendications

1. Canule pour ponction comprenant un corps tubulaire (1) dont l'une des extrémités comporte une avancée se terminant par une pointe (2) permettant d'inciser le matériau à ponctionner, la pointe (2) étant reliée au corps tubulaire (1) par une passerelle (4) constituant une surface lisse de guidage du matériau ponctionné vers le corps tubulaire, caractérisée en ce que la portion du corps tubulaire opposée à ladite passerelle est un secteur plein coopérant avec cette passerelle pour définir 2 ouvertures (5) situées de part et d'autre de la passerelle.

2. Canule pour ponction selon la revendication 1 caractérisée en ce que la passerelle (4) est reliée à la pointe (2) de la canule (1) dans une zone sensiblement centrée sur une génératrice du corps tubulaire.

3. Canule pour ponction selon l'une des revendications précédentes, caractérisée en ce que les ouvertures (5) comportent une taille en biais (5') dans leur zone périphérique.

4. Canule pour ponction selon l'une des revendications précédentes, caractérisée en ce que la passerelle (4) est reliée à la pointe (2) de la canule (1) par soudure.

5. Canule pour ponction selon l'une des revendications précédentes, caractérisée en ce que la passerelle (4) est infléchie vers l'intérieur de la canule (1).

6. Procédé de fabrication d'une canule pour ponction, caractérisé en ce que l'on exerce une force ponctuelle ou linéaire sur l'arête antérieure d'un matériau (1) pour canule de forme tubulaire, on enfonce ainsi le tube jusqu'à ce qu'il entre en contact avec la partie du tube radialement opposée, on fixe éventuellement le tube, on le taille de manière à former une passerelle (4) s'étendant de la pointe (2) de la canule (1) vers l'arrière avec deux ouvertures latérales (5).

7. Procédé de fabrication selon la revendication 6, caractérisé en ce que les ouvertures sont taillées en biais (5') et la partie non enfoncée du tube est taillée pour former des arêtes coupantes (3) se terminant en pointe (2), la distance entre les arêtes coupantes (3) étant plus faible que le diamètre externe du tube (1).

## Claims

1. Puncturing cannula comprising a tubular body (1) one of whose ends comprises a projection ending in a point (2) permitting incision of the substance to be punctured, the point (2) being connected to the tubular body (1) by a bridge (4) constituting a smooth surface for guiding the punctured substance towards the tubular body, characterised in that the portion of the tubular body opposite the said bridge is a full sector cooperating with this bridge so as to define 2 openings (5) situated on each side of the bridge.

2. Puncturing cannula according to Claim 1, characterised in that the bridge (4) is connected to the point (2) of the cannula (1) in a zone substantially centred on a generatrix of the tubular body.

3. Puncturing cannula according to one of the preceding claims, characterised in that the openings (5) comprise a slanting cut (5') in their peripheral zone.

4. Puncturing cannula according to one of the preceding claims, characterised in that the bridge (4) is connected to the point (2) of the cannula (1) by welding.

5. Puncturing cannula according to one of the preceding claims, characterised in that the bridge (4) is bent in towards the inside of the cannula (1).

6. Method for manufacturing a puncturing cannula, characterised in that a punctiform or linear force is exerted on the front edge of a cannula material (1) of tubular shape, the tube is thus pushed in until it comes into contact with the radially opposite part of the tube, the tube is fixed if appropriate, and it is cut in such a way as to form a bridge (4) extending from the point (2) of the cannula (1) towards the rear with two lateral openings (5).

7. Manufacturing method according to Claim 6, characterised in that the openings are cut with a slant (5') and the part of the tube not pushed in is cut so as to form cutting edges (3) ending in a point (2), the distance between the cutting edges (3) being smaller than the external diameter of the tube (1).

## Patentansprüche

1. Kanüle zur Punktion, umfassend einen röhrenförmigen Körper (1), dessen eines Ende einen Vorsprung aufweist, der in einer Spitze (2) endet, die das Einschneiden des zu punktierenden Materials erlaubt, wobei die Spitze (2) mit dem röhrenförmigen Körper (1) über einen Steg (4) verbunden ist, der eine glatte Oberfläche zur Führung des punktierten Materials gegen den röhrenförmigen Körper aufweist, **dadurch gekennzeichnet, daß** der Abschnitt des röhrenförmigen Körpers, der dem Steg gegenüberliegt, ein voller Sektor ist, der mit dem Steg zusammenwirkt, um zwei Öffnungen (5) zu begrenzen, die auf beiden Seiten von dem Steg liegen.

2. Kanüle nach Anspruch 1, dadurch gekennzeichnet, daß der Steg (4) mit der Spitze (2) der Kanüle (1) in einem Bereich im wesentlichen in der Mitte auf einer Mantellinie des röhrenförmigen Körpers verbunden ist.

3. Kanüle nach einem der vorhergehenden Ansprüche, dadurch gekennzeichnet, daß die Öffnungen (5) angeschrägte Schnittkanten (5') an ihrem Randbereich aufweisen.

4. Kanüle nach einem der vorhergehenden Ansprüche, dadurch gekennzeichnet, daß der Steg (4) mit der Spitze (2) der Kanüle (1) durch Löten bzw. Schweißen verbunden ist.

5. Kanüle nach einem der vorhergehenden Ansprüche, dadurch gekennzeichnet, daß der Steg (4) zum Inneren der Kanüle (1) gebogen ist.

6. Verfahren zur Herstellung einer Kanüle zur Punktion, dadurch gekennzeichnet, daß eine punktuelle oder lineare Kraft auf eine vordere Kante eines Materials (1) für eine röhrenförmige Kanüle ausgeübt wird, das Rohr so weit eingedrückt wird, bis es in Berührung mit dem radial gegenüberliegenden Teil des Rohres tritt, das Rohr eventuel' fixiert wird und es geschnitten wird, um einen Steg (4) zu bilden, der sich von der Spitze (2) der Kanüle (1) nach hinten mit zwei seitlichen Öffnungen (5) erstreckt.

7. Verfahren zur Herstellung nach Anspruch 6, dadurch gekennzeichnet, daß die Öffnungen schräg (5') geschnitten sind und der nicht eingedrückte Teil des Rohres geschnitten wird, um Schneidekanten (3) zu bilden, die an der Spitze (2) enden, wobei der Abstand zwischen den Schneidekanten (3) geringer ist als der äußere Durchmesser des Rohres (1).

EP 0 238 419 B1

Fig.1.

Fig.2.

5

Fig.4.

Fig.3.